# EUROPÄISCHE PATENTSCHRIFT

(11) **EP 0 468 320 B1**
(45) Veröffentlichungstag und Bekanntmachung des Hinweises auf die Patenterteilung: **22.03.1995**
(21) Anmeldenummer: 91111701.8
(22) Anmeldetag: 13.07.1991
(51) Int. Cl.: C07C 31/20, C07C 29/00

(54) **Verfahren zur Herstellung von Glykolen aus Formaldehyd**
Process for the preparation of glycols from formaldehyde
Procédé de préparation de glycols à partir de la formaldéhyde

(30) Priorität: 21.07.1990 DE 4023255
(43) Veröffentlichungstag der Anmeldung: 29.01.1992
(73) Patentinhaber: BASF Aktiengesellschaft, 67063 Ludwigshafen (DE)
(72) Erfinder: Gehrer, Eugen, Dr., W-6700 Ludwigshafen (DE); Harder, Wolfgang, Dr., W-6940 Weinheim (DE)

(56) Entgegenhaltungen:
- EP-A- 0 000 912
- EP-A- 0 007 102
- EP-A- 0 036 511
- FR-A- 656 178

## Beschreibung

Die Erfindung betrifft ein Verfahren zur zweistufigen Herstellung von Glykolen, insbesondere Propylenglykol aus Formaldehyd, wobei in einer ersten Stufe der Formaldehyd zu Polyhydroxyverbindungen ("Formose") kondensiert und in einer zweiten Stufe die Formose einer katalytischen Hydrogenolyse unterworfen wird.

Propylenglykol wird technisch nahezu ausschließlich durch Hydrolyse von Propylenoxid hergestellt. Die Synthese von Propylenoxid ist aber aufwendig oder im Falle der Chlorhydrinsynthese mit der Schwierigkeit der Entsorgung von umweltbelastenden Nebenprodukten behaftet.

Es hat daher nicht an Versuchen gefehlt, ausgehend von anderen Ausgangsmaterialien Propylenglykol zu gewinnen.

So wurde gemäß zahlreichen Literaturstellen (z.B. J.A.C.S. 54, 4116-4117 (1932), US 2 983 734, US 2 749 371, US 3 538 019, US 2 868 847, FR 2 603 276, US 3 055 840, US 3 963 788, US 3 963 789 oder US 4 430 253) die Hydrogenolyse von Zuckern zu Gemischen von Ethylenglykol, Propylenglykol und Glycerin untersucht, ohne daß es zu einer industriellen Verwertung dieser Reaktion kam.

Ferner wurde schon die Möglichkeit der unmittelbaren Herstellung von Propylenglykol aus Formaldehyd beschrieben. So erhält man z.B. gemäß US 4 496 781, US 4 565 896 oder JP 61 053 235 durch Hydroformylierung (Umsatz mit CO/H₂) und Hydrierung unter hohem Druck mit geringen Raum-Zeitausbeuten Gemische von Polyhydroxyverbindungen, die auch Glycole enthalten.

Aus DE 31 34 046, EP 71 458, EP 71 457, EP 147 983 und US 4 412 085 ist ferner bekannt, daß durch radikalische Addition von Methanol an Formaldehyd Ethylenglycol entsteht. Dieses Verfahren ist jedoch aufgrund des hohen Bedarfs an teuren Radikalstartern unwirtschaftlich. Auf photochemischem weg lassen sich nach JP 59 220 638 beim Bestrahlen von wäßrigen Formaldehydlösungen geringe Mengen an Ethylenglycol und Propylenglycol erhalten. Schließlich entstehen gemäß DE 30 18 844 bei der elektrochemischen Kupplung von Formaldehyd neben Ethylenglycol auch geringe Mengen an Propylenglycol. Alle diese verfahren lieferten bisher nur unbefriedigende Ausbeuten an Glycolen und die Reaktionsprodukte enthielten nur sehr geringe Mengen des gewünschten Propylenglykols.

FR-A 656 178 betrifft ein Verfahren zur Herstellung von Gemischen ein- und mehrwertiger Alkohole durch die Hydrierung von Formoselösungen, erhalten durch die Kondensation von Formaldehyd in Gegenwart einer Base, wobei der basische Katalysator aus der Formosebildungsreaktion (Formoin-Reaktion) während der Hydrierung im erhaltenen Reaktionsgemisch verbleibt, d.h. die Hydrierung der im Formosegemisch enthaltenen Carbonylgruppen zu Hydroxygruppen wird in basischem Medium durchgeführt. Dies hat den Nachteil, daß es im Zuge der Hydrierung durch basenkatalysierte Aldolkondensationen zur zusätzlichen Bildung erheblicher Mengen an höheren Aldolkondensationsprodukten und teerartigen Produkten kommt. Weiterhin wird nur eine Hydrierung der in der Formose enthaltenen Carbonylgruppen zu Hydroxygruppen vorgenommen, jedoch keine Hydrogenolyse, d.h. es wird lediglich Formit erzeugt.

Gemäß EP-A 7102 wird die basische Formoselösung zunächst unter basischen Bedingungen hydriert, jedoch nur bis zu einem Teilumsatz, nämlich bis der Gehalt an reduzierbaren Gruppen (bestimmt als Carbonylgruppen) unter 1,5 Gew.-% gesunken ist. Erst danach wird der basische, aus der Formoin-Reaktion stammende Katalysator abgetrennt, die teilhydrierte Lösung angesäuert und in einer zweiten Stufe zu Ende hydriert. Eine Hydrogenolyse wird nicht vorgenommen. Dementsprechend wird im Verfahren von EP-A 7102 lediglich eine Formitlösung erhalten, die aus einer unüberschaubaren Vielzahl verschiedener Polyole besteht.

Es bestand daher die Aufgabe, Propylenglykol aus leicht zugänglichen Ausgangsmaterialien in einfacher Weise herzustellen. Diese Aufgabe wurde mit der vorliegenden Erfindung gelöst, mit einem Verfahren zur Herstellung von Propylenglykol und Ethylenglykol aus Formaldehyd, wobei man in einer ersten Stufe Formaldehyd in an sich bekannter Weise in alkalischem Medium durch Selbstkondensation in ein Gemisch von Polyhydroxyverbindungen überfuhrt, vor der katalytischen Hydrogenolyse die Carbonyl- und Acetalgruppen von Verbindungen des Polyhydroxyverbindungsgemisches katalytisch hydriert und dieses Gemisch einer katalytischen Hydrogenolyse unterwirft, bei der ein Wasserstoffverbrauch von mindestens 0,5 Mol Wasserstoff pro Mol zur Erzeugung der Polyhydroxyverbindungen eingesetztem Formaldehyd eingestellt wird.

Der glatte Verlauf dieser Methode war überraschend, da das durch Selbstkondensation von Formaldehyd erhältliche Formosegemisch mehr als 40 Hauptverbindungen enthält, unter denen sich neben Glucose, Fructose oder Xylose z.B. auch unnatürliche verzweigte Polyhydroxyverbindungen befinden. Dies bedeutet, daß die Vielfalt der bei der Formosereaktion entstehenden Stoffe wieder zu einigen wenigen Wertprodukten zusammengeführt wird und man somit durch eine zweistufige Reaktion mit hoher Ausbeute Formaldehyd in Propylenglykol und Ethylenglykol umwandeln kann.

Die Formosereaktion ist schon sehr lange aus Ann. 120, 295 (1861) und J. Pr. Chem. 33, 321 (1886) und aus neuerer Literatur aus Pfeil, Chem. Ber. 84, 229 (1951) G. Harsch, Z. Naturforsch. 38b 1257-1268 (1983) bekannt, ohne daß sie bisher zu einer gewerblichen Verwertung geführt hätte.

Zur Ausführung des erfindungsgemäßen Verfahrens geht man im einzelnen zweckmäßigerweise wie folgt vor:
a) Formosereaktion
   Die Herstellung der Formoselösung kann in Anlehnung an eine Reihe von Methoden wie sie z.B. in Alvin Weiss, J. Catal. 48, 354-364 (1977), T. Mizuno, J. Agr. Chem. Soc. Jap. 44, No. 7, 324-331 (1970) und Y. Shigemasa, Bull. Chem. Soc. Jap. 50 (6), 1527-31 (1977) beschrieben sind und auf die hiermit Bezug genommen wird, durchgeführt werden.
   In der Regel werden basische Katalysatoren, wie CaO, SrO, BaO, TlOH, PbO oder auch organische Basen sowie Thiazolium- oder Imidazoliumsalze verwendet. Man kann die Formosereaktion kontinuierlich, ansatzweise oder auch semikontinuierlich durchführen. Der Formaldehyd kann in Lösung, gasförmig oder in Form von Paraformaldehyd oder Trioxan zugeführt werden. Die Reaktionstemperaturen liegen zwischen Raumtemperatur und 110°C. Der pH-Wert liegt je nach Katalysatorsystem zwischen 5 und 12.
b) Katalytische Hydrogenolyse
   Das entstandene rohe Formosegemisch wird zweckmäßig direkt der Hydrogenolyse zugeführt. In vielen Fällen ist es jedoch vorteilhaft, den für die Selbstkondensation des Formaldehydes eingesetzten Katalysator vorher abzutrennen. Im Falle der Hydroxide von Ca, Sr, Ba oder Pb kann man z.B. den Katalysator leicht durch Fällen mit H₂SO₄ abtrennen.
   Nach einer bevorzugten Ausführungsform wird ferner vor der Hydrogenolyse eine Reduktion der Carbonylgruppen bzw. Acetalgruppen der in der Formose enthaltenen Verbindungen unter milden Bedingungen, im neutralen oder sauren Bereich, vorgeschaltet, um Nebenreaktionen, wie Verharzungen und die Bildung von Milchsäure oder schwer reduzierbarer Zuckersäuren zu verringern. Diese reduzierte Formose wird im folgenden Formit genannt.
   Für diese Hydrierung eignen sich grundsätzlich sämtliche Hydrierkatalysatoren für Carbonylverbindungen. Besonders bevorzugt läßt sich diese Reaktion mit Ru- oder Ni-Katalysatoren bei 100 bis 150°C und einem Druck von 20 bis 200 bar H₂ durchführen.
   Zur Erreichung einer hochselektiven Bildung von Ethylenglykol und Propylenglykol bei der, gegebenenfalls nach der vorgeschalteten Hydrierung, stattfindenden Hydrogenolyse des komplexen, aus über 40 verschiedenen, geradkettigen und verzweigten Polyhydroxyverbindungen bestehenden Formosegemisches ist es erforderlich, daß dieses bei der Umsetzung mit Wasserstoff insgesamt, d.h. im Zuge der Hydrierung und Hydrolyse, mindestens 0,5 Mol Wasserstoff pro Mol zur Erzeugung des Formosegemisches eingesetztem Formaldehyd aufnimmt. Die Wasserstoffaufnahme des Formosegemisches kann anhand üblicher Druckmeß- und Druckregelungsventile bei Anwendung der im folgenden angegebenen Reaktionsbedingungen auf einfache Weise kontrolliert und gesteuert werden.
   Die Hydrogenolyse des Formosegemisches oder des Formitgemisches erfolgt bei 50 bis 300°C, insbesondere bei 100 bis 250°C, bevorzugt bei 180 bis 220°C und bei Wasserstoffdrücken von 1 bis 1000 bar, vorzugsweise 10 bis 600 bar und insbesondere 40 bis 100 bar, mit homogenen oder heterogenen Hydrierkatalysatoren. Bevorzugt werden Cu- Ni- oder Ru-haltige Katalysatoren auf Trägern oder als Suspension (Raney-Ni) verwendet. Es können im niederen Temperaturbereich auch homogene Katalysatoren, wie Ru(PAr₃)₄, eingesetzt werden.
   Das Reaktionsgemisch der katalytischen Hydrogenolyse wird mit an sich bekannten Methoden der Stofftrennung aufgearbeitet. Zweckmäßigerweise wird zuerst das Wasser abgetrennt und anschließend das Glykolgemisch einer Feindestillation unterworfen. Die bei der Reaktion als Nebenprodukt anfallende Milchsäure kann nach an sich bekannten Methoden (wie sie z.B. in EP 175 558 beschrieben sind) zu einer weiteren Fraktion Propylenglykol hydriert werden.

### Beispiel

a) Formoseherstellung
Anfahren der Reaktion:
In einem 100 ml Kolben mit aufgesetztem Rückflußkühler, pH-Elektrode, Thermometer, Formaldehyd-Lösung- und CaO/H₂O-Zulauf werden 40 g Fructose und 60 g Wasser vorgelegt und auf 90°C erwärmt. Anschließend dosiert man 4 g/min einer 5 %igen Suspension von CaO in Wasser bis sich ein pH-Wert von 9.8 einstellt. Die Lösung wird dabei gelbbraun. Nun startet man die Zugabe von 5 g/min 37 %igem Formaldehyd. Nachdem die Reaktion angesprungen ist (die Reaktion ist exotherm und die Reaktionsmischung kann zu kochen beginnen) senkt man die Temperatur langsam auf 60°C ab.
Stationärer Betrieb:
Bei 60°C werden 5 g/min 37 %ige Formaldehyd-Lösung und 4 g/min CaO (5 %) zudosiert. Es stellt sich ein pH-Wert von 8 bis 10 ein. Die Lösung ist hellgelb. Sie enthält nach dem Verlassen des Reaktors noch etwas Restformaldehyd, der beim Lagern bei Raumtemperatur vollständig abreagiert. Dieser stationäre Betrieb ist sehr stabil.
Aufarbeitung:
Für die anschließende Hydrierung wird das Calcium mit Schwefelsäure ausgefällt und ein pH-Wert von 3 eingestellt. Für 1 l Formose benötigt man ca. 196 ml 20 %ige Schwefelsäure. Das System puffert bei einem pH von ca. 3.5 und das Calciumsulfat fällt erst nach Zugabe von 3/4 der erforderlichen Menge Schwefelsäure aus.
Analytik:
Beim Eindampfen der salzfreien, sauren Formose unter vermindertem Druck bei 60°C erhält man 100 bis 105 % organischen Rückstand mit ca. 6 % fest gebundenem Wasser. Die Ausbeute an organischen Produkten liegt somit bei ca. 97 %. Es ist eine hellgelbe, zähe, nicht mehr fließfähige, klebrige Masse. Mittels Dinitrophenylhydrazinderivat wurde der Restgehalt von Formaldehyd (einschließlich des acetalgebundenen Formaldehyds) auf 0.001 % bestimmt. Die GC-Analyse der Trimethylsilylderivate zeigt ein Gemisch von Polyhydroxyverbindungen mit bevorzugt 4 bis 7 Kohlenstoffatomen im Molekül.
b) Hydrierung des Formose-Gemisches zu Formit
In einem 300 ml Autoklaven werden 60 ml der gemäß a) hergestellten aber nicht eingedampften sauren und salzfreien Formose und 30 g Katalysator (5 % Ru auf Kohle) bei einem Wasserstoffdruck von 90 bar unter Rühren 30 Minuten bei 120°C hydriert. Die Reaktion beginnt schon währen des Aufheizens (Wasserstoffverbrauch). Man erhält auch nach mehrmaliger Verwendung desselben Katalysators eine wäßrige Polyollösung (Formit) die praktisch frei von Carbonyl- und Acetalgruppen ist.
c) Hydrogenolyse
In einem 300 ml Autoklaven werden 60 ml der gemäß b) hergestellten Formitlösung, 3 g CaO und 30 g (Trockengewicht) Katalysator (5 % Ru auf Kohle mit Na₂S 1:1 vergiftet - zur Unterdrückung der Methanisierungsreaktion) unter einem Wasserstoffdruck von 90 bar unter Rühren 2 Stunden bei 200°C hydrogenolysiert. Das Filtrat des Reaktionsgemisches enthält (ermittelt durch GC-Analyse der Trimethylsilylderivate) bezogen auf den eingesetzten Formaldehyd nach mehrmaliger (6 bis 8 mal) Verwendung desselben Katalysators:

| mol-% | |
|---|---|
| 44.4 | Propylenglycol |
| 23.3 | Ethylenglycol |
| 8.3 | 1,2-Butylenglycol |
| 9.5 | 2,3-Butylenglycol |
| 10.3 | Milchsäure |
| 95.9 | Summe der Wertprodukte |

Bei der Hydrierung der Formoselösung zur Formitlösung und deren anschließender Hydrogenolyse wurden insgesamt 0,54 Mol Wasserstoff pro Mol zur Herstellung der Formose eingesetztem Formaldehyd verbraucht.

## Patentansprüche

1. Verfahren zur Herstellung von Propylenglykol und Ethylenglykol aus Formaldehyd, dadurch gekennzeichnet, daß man in einer ersten Stufe Formaldehyd in an sich bekannter Weise in alkalischem Medium durch Selbstkondensation in ein Gemisch von Polyhydroxyverbindungen überführt, vor der katalytischen Hydrogenolyse die Carbonyl- und Acetalgruppen von Verbindungen des Polyhydroxyverbindungsgemisches katalytisch hydriert und dieses Gemisch einer katalytischen Hydrogenolyse unterwirft, bei der ein Wasserstoffverbrauch von mindestens 0,5 Mol Wasserstoff pro Mol zur Erzeugung der Polyhydroxyverbindungen eingesetztem Formaldehyd eingestellt wird.

2. Verfahren gemäß Anspruch 1, dadurch gekennzeichnet, daß man zum Zwecke der katalytischen Hydrogenolyse eine heterogene katalytische Hydrierung bei Temperaturen von 50 bis 300°C und Wasserstoffdrücken von 10 bis 600 bar durchführt.

3. Verfahren gemäß Anspruch 2, dadurch gekennzeichnet, daß man die katalytische Hyrogenolyse in Gegenwart von Calciumoxid durchführt.

4. Verfahren gemäß Anspruch 1, dadurch gekennzeichnet, daß man die Selbstkondensation des Formaldehyds bei pH-Werten von 8 bis 10 unter Verwendung von Calciumoxid als Katalysator durchführt.

5. Verfahren gemäß Anspruch 1, dadurch gekennzeichnet, daß man die Hyrierung bei Temperaturen von 100 bis 150°C und Wasserstoffdrücken von 20 bis 200 bar an Ruthenium- oder Nickelkatalysatoren durchführt.

6. Verfahren gemäß Anspruch 1, dadurch gekennzeichnet, daß man Propylenglykol und Ethylenglykol aus dem Reaktionsgemisch durch Destillation oder Extraktivdestillation gewinnt.

## Claims

1. A process for the preparation of propylene glycol and ethylene glycol from formaldehyde, which comprises, in a first step, converting formaldehyde into a mixture of polyhydroxyl compounds in a conventional manner in an alkaline medium by self-condensation, subjecting the carbonyl and acetal groups of compounds of the polyhydroxyl compound mixture to catalytic hydrogenation before the catalytic hydrogenolysis, and subjecting this mixture to catalytic hydrogenolysis with a hydrogen consumption of at least 0.5 mol of hydrogen per mol of formaldehyde employed for forming the polyhydroxyl compounds.

2. A process as claimed in claim 1, wherein the catalytic hydrogenolysis is effected by heterogeneous catalytic hydrogenation at from 50 to 300°C and a hydrogen pressure of from 10 to 600 bar.

3. A process as claimed in claim 2 , wherein the catalytic hydrogenolysis is carried out in the presence of calcium oxide.

4. A process as claimed in claim 1, wherein the self-condensation of the formaldehyde is carried out at a pH of from 8 to 10 using calcium oxide as catalyst.

5. A process as claimed in claim 1, wherein the hydrogenation is carried out at from 100 to 150°C and a hydrogen pressure of from 20 to 200 bar on a ruthenium or nickel catalyst.

6. A process as claimed in claim 1, wherein propylene glycol and ethylene glycol are isolated from the reaction mixture by distillation or extractive distillation.

## Revendications

1. Procédé de fabrication du propylèneglycol et de l'éthylèneglycol à partir du formaldéhyde, caractérisé en ce que, au cours d'une première étape, on convertit le formaldéhyde de manière en soi connue et en milieu alcalin par autocondensation en un mélange de composés polyhydroxylés, avant l'hydrogénolyse catalytique, on hydrogène catalytiquement les radicaux carbonyle et acétal de composés du mélange des composés polyhydroxylés et on soumet ce mélange à une hydrogénolyse catalytique, au cours de laquelle on établit une consommation d'hydrogène d'au moins 0,5 mole d'hydrogène par mole du formaldéhyde mis en oeuvre pour l'obtention des composés polyhydroxylés.

2. Procédé suivant la revendication 1, caractérisé en ce que, dans le but de l'hydrogénolyse catalytique, on entreprend une hydrogénation catalytique hétérogène à des températures de 50 à 300°C et sous des pressions d'hydrogène de 10 à 600 bars.

3. Procédé suivant la revendication 2, caractérisé en ce que l'on entreprend l'hydrogénolyse catalytique en présence d'oxyde de calcium.

4. Procédé suivant la revendication 1, caractérisé en ce que l'on entreprend l'autocondensation du formaldéhyde à des valeurs de pH de 8 à 10 et en recourant à l'emploi d'oxyde de calcium à titre de catalyseur.

5. Procédé suivant la revendication 1, caractérisé en ce que l'on entreprend l'hydrogénation à des températures de 100 à 150°C et sous des pressions d'hydrogène de 20 à 200 bars, sur des catalyseurs au ruthénium ou au nickel.

6. Procédé suivant la revendication 1, caractérisé en ce que l'on recueille le propylèneglycol et l'éthylèneglycol à partir du mélange réactionnel par distillation ou distillation extractive.
